(19) Europäisches Patentamt
European Patent Office
Office européen des brevets

(11) **EP 2 329 880 B1**

(12) **EUROPEAN PATENT SPECIFICATION**

(45) Date of publication and mention
of the grant of the patent:
**31.08.2016 Bulletin 2016/35**

(51) Int Cl.:
**B01J 23/88** (2006.01)    **C07C 27/14** (2006.01)
**C07C 45/35** (2006.01)    **C07C 47/22** (2006.01)
**C07C 51/25** (2006.01)    **C07C 57/05** (2006.01)

(21) Application number: **09817710.8**

(22) Date of filing: **25.09.2009**

(86) International application number:
**PCT/JP2009/066657**

(87) International publication number:
**WO 2010/038677 (08.04.2010 Gazette 2010/14)**

(54) **CATALYST FOR PRODUCING ACROLEIN AND/OR ACRYLIC ACID AND PROCESS FOR PRODUCING ACROLEIN AND/OR ACRYLIC ACID USING THE CATALYST**

KATALYSATOR ZUR ACROLEIN- UND/ODER ACRYLSÄUREHERSTELLUNG UND VERFAHREN ZUR ACROLEIN- UND/ODER ACRYLSÄUREHERSTELLUNG MITHILFE DIESES KATALYSATORS

CATALYSEUR POUR LA PRODUCTION D ACROLÉINE ET/OU D ACIDE ACRYLIQUE ET PROCÉDÉ DE PRODUCTION D ACROLÉINE ET/OU D ACIDE ACRYLIQUE UTILISANT LE CATALYSEUR

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **30.09.2008 JP 2008252084**

(43) Date of publication of application:
**08.06.2011 Bulletin 2011/23**

(73) Proprietor: **Nippon Shokubai Co., Ltd.**
**Osaka-shi, Osaka 541-0043 (JP)**

(72) Inventors:
• **TANIMOTO, Michio**
**Himeji-shi**
**Hyogo 671-1214 (JP)**

• **HAKOZAKI, Nobuyuki**
**Himeji-shi**
**Hyogo 671-1143 (JP)**

(74) Representative: **Müller-Boré & Partner**
**Patentanwälte PartG mbB**
**Friedenheimer Brücke 21**
**80639 München (DE)**

(56) References cited:
**EP-A1- 1 714 955    EP-A1- 2 177 500**
**EP-A2- 1 571 137    JP-A- 9 057 106**
**JP-A- 53 048 095**

## Description

The present invention relates to a catalyst suitable for producing acrolein and/or acrylic acid from propylene by gas-phase catalytic oxidation with molecular oxygen and a process for producing acrolein and/or acrylic acid using the catalyst.

Acrylic acid is industrially important as a raw material for various synthetic resins, paints and plasticizers, and recently, its importance as a raw material for an absorbent polymer has been increasing. The most common process for producing acrylic acid is a two-stage oxidation process in which acrolein is mainly produced from propylene by gas-phase catalytic oxidation and then acrylic acid is produced from the obtained acrolein by gas-phase catalytic oxidation.

Upon the process for producing acrylic acid by gas-phase catalytic oxidation of propylene in the presence of molecular oxygen-containing gas, an oxidation catalyst is used for producing acrolein and/or acrylic acid from propylene in the above first stage. However, the catalytic performance such as an yield of acrolein, catalytic life and the like, of this catalyst is not necessarily satisfactory, and various modifications centered around a molybdenum-bismuth-containing catalyst have been made by many companies for the purpose of improving catalyst performance.

For example, Patent literature 1 discloses a catalyst which has crystalline phases of $\beta$-$X^1MoO_4$ mainly and $Fe_2(MoO_4)_3$ secondly and does not contain $MoO_3$. Patent literature 2 discloses a catalyst consisting of cobalt, molybdenum, bismuth and iron, and having a crystalline phase expressed by $Bi_2Fe_2Mo_2O_{12}$ in its active component. Patent literature 3 discloses a method for preparing a catalyst containing molybdenum, bismuth and iron as essential elements, in which dry-powdered iron molybdate gel ($Fe(MoO_4)_3$) is used for preparing the catalyst. Patent literature 4 discloses that, in a catalyst containing molybdenum, bismuth and iron as essential elements, by heating the catalyst with reduced catalytic activity under specific conditions, a peak attributed to a crystalline phase $Bi_2Fe_2Mo_2O_{12}$ in a catalytic active component reduces whereas a peak attributed to $Fe_2(MoO_4)_3$ is regrown, resulting in activation of the catalyst performance.

PATENT LITERATURE 1
Japanese Unexamined Patent Application Publication No. 2000-169149
PATENT LITERATURE 2
Japanese Examined Patent Application Publication No. 56-28180
PATENT LITERATURE 3
Japanese Unexamined Patent Application Publication No. 1-168344
PATENT LITERATURE 4
Japanese Unexamined Patent Application Publication No. 63-137755

EP 1 571 137 A2 discloses a catalyst for catalytic gas phase oxidation of acrolein with molecular oxygen to produce acrylic acid, the catalyst comprising molybdenum and vanadium and further comprising a volatile catalyst poison ingredient in an amount of 10 to 100 ppb by mass as measured by ion chromatography. Further, EP 1 714 955 A1 discloses a fixed bed reactor for gas-phase catalytic oxidation, which comprises a reaction tube filled with a gas-phase oxidation catalyst, wherein a solid acid, of which acid strength (Ho) meets an inequality: $-5.6 \leq H_0 \leq 1.5$, is placed in a gas passage containing a starting material compound and/or a produced compound. EP 2 177 500 A1, which represents prior art according to Art. 54(3) EPC, discloses a process for producing acrylic acid by two-stage catalytic vapor-phase oxidation method comprising catalytic vapor-phase oxidation of a propylene-containing gas at a first fixed bed reactor which is loaded with a catalyst for converting propylene to acrolein by catalytic vapor-phase oxidation, to produce an acrolein-containing gas, and catalytic vapor-phase oxidation of the formed reaction gas at a second fixed bed reactor which is loaded with a catalyst for converting acrolein to acrylic acid by catalytic vapor-phase oxidation, to produce acrylic acid, wherein the catalyst to be loaded in the second fixed bed reactor for converting acrolein to acrylic acid by catalytic vapor-phase oxidation is one containing molybdenum and vanadium represented by the specific general formula $Mo_hV_iW_jY1_kY2_lY3_mY4_nO_y$.

Acrylic acid is produced as much as several millions of tons per year worldwide, and in addition, the demand therefor as a raw material for an absorbent polymer is still increasing. Further, due to recent jumps in prices of the raw material propylene, enhancing the yield of acrylic acid in an industrial scale by just 0.1 % would have a significant meaning in terms of economy. Upon the process for producing acrolein and/or acrylic acid from propylene, that is the above first stage, enhancement of its yield is desired. Although the catalysts disclosed in Patent literatures 1 to 4 are somewhat improved in the intended catalyst performance such as the yield of acrolein and/or acrylic acid, catalyst life and the like, there is still room for improvement in an industrial scale.

In the catalyst disclosed in Patent literature 1, a space-time yield of acrolein and acrylic acid is relatively high; however, it is evaluated under a condition of a low raw material propylene concentration of 5.5%. Further, even under the condition where the raw material propylene concentration is low, the reaction temperature must be increased by as much as 10°C after only 2000 hours of operation, and thus, the suppression of performance deterioration with time is insufficient and there is unknown about the catalyst performance when the reaction is continued for a long period. The catalyst described in the Patent literature 2 has an acrylic acid selectivity of at most about 88% in an initial stage of the reaction, which is insufficient in respect of the yield of acrylic acid. In addition, the catalyst life is not evaluated and there is unknown about the catalyst performance when acrylic acid is continuously produced over a long period. In Example of Patent literature 3, though it is disclosed that the yield of acrylic acid is improved in some extent, it is a result of being reacted in a laboratory-scale reaction tube under a condition of a low raw material propylene concentration of 4.5%. In addition, the catalyst life is not evaluated and there is unknown about the practical catalyst performance. Patent literature 4 merely discloses a method for reactivating of a well-known catalyst and does not teach a new catalyst for the purpose of improving the catalyst performance. Furthermore, none of Patent literatures 1 to 4 discloses a technical idea of enhancing the yields of acrolein and/or acrylic acid over a long period by controlling a crystallinity of a catalytic active component within an appropriate range.

The present invention has been achieved in view of the above circumstances, and an object of the present invention is to provide a catalyst which is excellent in catalyst performance such as catalytic activity and selectivity as well as in catalyst life and provides stable performance over a long period in producing acrolein and/or acrylic acid from propylene.

The catalyst of the present invention which solves the above problems is a catalyst for producing acrolein and/or acrylic acid from propylene by gas-phase catalytic oxidation with molecular oxygen-containing gas, comprising: a catalytic active component, wherein: the catalyst is prepared by a method comprising the step of adding an aqueous solution containing cobalt and bismuth to an aqueous solution containing molybdenum over a period of 30 seconds to 10 minutes; the catalytic active component has a composition expressed by the following formula (4); and

the catalytic active component contains a crystalline part showing a peak at $2\theta = 26.5 \pm 0.5°$, measured by an X-ray diffraction analysis with Cu-K$\alpha$ radiation, and has a crystallinity T of 4% or more and 18% or less in the $2\theta$ range of 5° to 90°, measured by an X-ray diffraction analysis with Cu-K$\alpha$ radiation; and

a crystallinity ratio R expressed by the following formula (1) is in the range of 0.03 or more and 0.20 or less

$$R = M/T \qquad (1)$$

wherein M represents a crystallinity of the peak at $2\theta = 26.5 \pm 0.5°$ in the catalytic active component, measured by the X-ray diffraction analysis with Cu-K$\alpha$ radiation,

$$Mo_aBi_bCo_cX1_dX2_eX3_fX4_gO_z \qquad (4)$$

wherein Mo is molybdenum; Bi is bismuth; Co is cobalt; X1 represents at least one kind of element selected from the group consisting of iron and nickel; X2 represents at least one kind of element selected from the group consisting of alkali metals, alkali earth metals, boron and thallium; X3 represents at least one kind of element selected from the group consisting of tungsten, silicon, aluminum, zirconium and titanium; X4 represents at least one kind of element selected from the group consisting of phosphorus, tellurium, antimony, tin, cerium, lead, niobium, manganese, arsenic and zinc; O is oxygen; a, b, c, d, e, f, g and z mean atomic ratios of Mo, Bi, Co, X1, X2, X3, X4 and O, respectively, and meet inequalities: $a = 12$, $0.1 \le b \le 10$, $0.1 \le c \le 20$, $0.1 \le d \le 20$, $0.001 \le e \le 10$, $0 \le f \le 30$ and $0 \le g \le 10$, respectively; and z is a numeral value determined by oxidation states of respective elements. According to the catalyst of the present invention, since the crystallinity T is within the aforementioned range, the catalyst is excellent in catalytic performance such as catalytic activity and selectivity as well as in catalyst life, and provides stable performance over a long period.

When the crystallinity ratio R is adjusted in that range in addition to the crystallinity T, it becomes easy to obtain a catalyst for producing acrolein and/or acrylic acid which is more excellent in catalyst performance and catalyst life and provides stable performance over a long period.

In addition, the process for producing acrolein and/or acrylic acid of the present invention is a process for producing acrolein and/or acrylic acid by gas-phase catalytic oxidation of propylene with molecular oxygen, comprising the step of conducting the gas-phase catalytic oxidation in the presence of the catalyst of the present invention. According to the process of the present invention, it is possible to stably produce acrolein and/or acrylic acid at a high yield for a long period.

According to the catalyst and the process for producing acrolein and/or acrylic acid of the present invention, it is possible to stably produce acrolein and/or acrylic acid at a high yield for a long period in producing acrolein and/or acrylic acid by gas-phase catalytic oxidation of propylene with molecular oxygen.

The catalyst for producing acrolein and/or acrylic acid of the present invention and the process for producing acrolein and/or acrylic acid of the present invention using the catalyst will hereinafter be explained in detail; however, the present invention is not limited to the following description, and can be put into practice after appropriate modification or variations within a range meeting the gist of the present invention in addition to the following embodiments.

The catalyst for producing acrolein and/or acrylic acid of the present invention is a catalyst for producing acrolein and/or acrylic acid by gas-phase catalytic oxidation of propylene with molecular oxygen-containing gas. A raw material gas for producing acrolein and/or acrylic acid contains at least propylene and molecular oxygen-containing gas. The molecular oxygen-containing gas is not particularly limited as long as it contains molecular oxygen, and for example, gas consisting of only molecular oxygen may be used, or air may be used, as the molecular oxygen-containing gas.

The catalyst of the present invention comprises a catalytic active component containing molybdenum, bismuth and cobalt as essential elements, and the catalytic active component has a crystallinity T of 4% or more and 18% or less in the $2\theta$ range of 5° to 90°, measured by an X-ray diffraction analysis with Cu-K$\alpha$ radiation. The crystallinity T is preferably 7% or more and 15% or less. In the catalyst of the present invention, since the catalytic active component has the crystallinity T of 4% or more and 18% or less in the $2\theta$ range of 5° to 90°, measured by an X-ray diffraction analysis with Cu-K$\alpha$ radiation, the catalyst is excellent in catalytic performance such as catalytic activity, selectivity and the like, as well as in catalyst life, and provides stable performance over a long period. Although the reason is not clear, when the crystallinity T is less than 4%, the catalyst has low selectivity for producing acrolein and/or acrylic acid, whereas when the crystallinity T is more than 18%, the catalyst life becomes short, resulting in early degradation in performance.

The crystallinity in the present invention can be obtained by using an X-ray diffraction apparatus following the method according to JIS K-0131 (an absolute method). Thus, the crystallinity T is calculated by obtaining an X-ray diffraction intensity of all crystalline part(s) in the $2\theta$ range of 5° to 90° and a total X-ray diffraction intensity in the $2\theta$ range of 5° to 90°, and obtaining the ratio of the X-ray diffraction intensity of the all crystalline part(s) to the total X-ray diffraction intensity, as expressed by the following formula (2). Here, the total X-ray diffraction intensity means an integral intensity of the X-ray diffraction in the $2\theta$ range of 5° to 90°, and the

X-ray diffraction intensity of the all crystalline part(s) means an integral intensity of the X-ray diffraction of the crystalline part(s) in the $2\theta$ range of 5° to 90°.

$$T = \text{(an X-ray diffraction intensity of the all crystalline part(s))/(a total X-ray diffraction intensity)} \times 100 \qquad (2)$$

The crystallinity T is obtained as follows, specifically. From a diffraction profile obtained by the X-ray diffraction analysis, a background is eliminated and then a halo pattern of an amorphous part is separated, thereby obtaining a diffraction profile of crystalline part(s). The diffraction profile of the crystalline part(s) is separated by peaks. An integral intensity ($S_n$) of the halo pattern of the amorphous part and integral intensities ($S_{c1}$, $S_{c2}$, $S_{c3}$ ...) of the respective peaks of the crystalline part(s) are obtained, and the X-ray diffraction intensity $S_c$ of all the crystalline part(s) is calculated from the sum of the integral intensities ($S_{c1} + S_{c2} + S_{c3} + ...$) of the respective peaks of the crystalline part(s). The crystallinity T is obtained from the formula: $T = S_c/(S_n + S_c) \times 100$.

In the catalyst of the present invention, a crystallinity ratio R, which is calculated as a ratio of a crystallinity M of the peak at $2\theta = 26.5 \pm 0.5°$ in the catalytic active component, measured by the X-ray diffraction analysis with Cu-K$\alpha$ radiation, to the crystallinity T, as expressed by the following formula (1), is in the range of 0.03 or more and 0.20 or less. Preferably, the crystallinity ratio is in the range of 0.05 or more and 0.13 or less.

$$R = M/T \qquad (1)$$

In the above formula (1), M represents a crystallinity of the peak at $2\theta = 26.5 \pm 0.5°$ when the catalytic active component is measured by the X-ray diffraction analysis with Cu-K$\alpha$ radiation. The M can be obtained by using an X-ray diffraction apparatus following the method according to JIS K-0131 (the absolute method). Thus, the M is calculated by obtaining an X-ray diffraction intensity of a crystalline part of the peak at $2\theta = 26.5 \pm 0.5°$ and a total X-ray diffraction intensity in the $2\theta$ range of 5° to 90°, and obtaining the ratio of the X-ray diffraction intensity at $2\theta = 26.5 \pm 0.5°$ to the total X-ray diffraction intensity, as expressed by the following formula (3). Here, the X-ray diffraction intensity of the crystalline part of the peak at $2\theta = 26.5 \pm 0.5°$ means an integral intensity of the X-ray diffraction having a peak at $2\theta = 26.5 \pm 0.5°$.

$$M = \text{(an X-ray diffraction intensity of a crystalline part of the peak)/(a total X-ray diffraction intensity)} \times 100 \qquad (3)$$

The crystallinity ratio R corresponds to the ratio of the X-ray diffraction intensity of the crystalline part of the peak at $2\theta = 26.5 \pm 0.5°$ to the X-ray diffraction intensity of all the crystalline part(s) in the $2\theta$ range of 5° to 90°. Thus, by defining the crystallinity ratio R, the ratio of the specific crystalline part in the whole crystalline part is defined, whereby a catalyst for producing acrylic acid which is more excellent in catalyst performance and catalyst life and provides stable performance over a long period can be easily obtained.

The catalyst for producing acrolein and/or acrylic acid of the present invention comprises the catalytic active component containing molybdenum, bismuth and cobalt as essential elements. For the catalyst for producing acrolein and/or acrylic acid of the present invention, it is important that the crystallinity T is within the aforementioned range, and the present invention can be applied to a catalyst which contains molybdenum, bismuth and cobalt essentially as the catalytic active component. As to the catalyst which contains molybdenum, bismuth and cobalt as essential elements, compositions of the catalytic active component and preparing methods are variously proposed by many companies; however, in the present invention, the catalyst comprises the catalytic active component having the composition expressed by the following formula (4). When the catalyst comprises the catalytic active component having the composition expressed by the following formula (4), the catalyst which is excellent in catalyst performance such as activity (i.e. conversion rate of propylene and yields of acrolein and/or acrylic acid), catalyst life and the like is easily obtained by adjusting the crystallinity T in an appropriate range:

$$Mo_aBi_bCo_cX1_dX2_eX3_fX4_gO_z \qquad (4)$$

wherein Mo is molybdenum; Bi is bismuth; Co is cobalt; X1 represents at least one kind of element selected from the group consisting of iron and nickel; X2 represents at least one kind of element selected from the group consisting of alkali metals, alkali earth metals, boron and thallium; X3 represents at least one kind of element selected from the group consisting of tungsten, silicon, aluminum, zirconium and titanium; X4 represents at least one kind of element selected from the group consisting of phosphorus, tellurium, antimony, tin, cerium, lead, niobium, manganese, arsenic and zinc; O is oxygen; a, b, c, d, e, f, g and z mean atomic ratios of Mo, Bi, Co, X1, X2, X3, X4 and O, respectively, and meet inequalities: $a=12$, $0.1 \leq b \leq 10$, $0.1 \leq c \leq 20$, $0.1 \leq d \leq 20$, $0.001 \leq e \leq 10$, $0 \leq f \leq 30$ and $0 \leq g \leq 10$, respectively; and z is a numeral value determined by oxidation states of respective elements.

The catalyst of the present invention may comprise an inert carrier for supporting the catalytic active component in addition to the catalytic component containing molybdenum, bismuth and cobalt as essential elements. Examples of the inert carrier include alumina, silica, silica-alumina, titania, magnesia, steatite, cordierite, silica-magnesia, silicon carbide, silicon nitride, zeolite, and the like. A shape of the carrier is not particularly limited, and a carrier having a spherical shape, a cylindrical shape, a ring shape, or the like, may be used.

A preparing method of the catalyst of the present invention is hereinafter explained. The preparing method of a catalyst is variously proposed; and the following method may be employed, for example.

As a raw material for the catalytic active component, an oxide, a hydroxide, or a salt (e.g. an ammonium salt, a nitrate salt, a carbonate salt, a sulfate salt, an organic acid salt or the like) of each element composing the catalytic active component, an aqueous solution or a sol thereof, a compound containing a plurality of elements, or the like, is mixed with water to give an aqueous solution (hereinafter referred to as a "mixed liquid of starting materials"), for example.

The obtained mixed liquid of starting materials is dried by a method such as heating or pressure reduction as required to obtain a catalyst precursor. For the drying method by heating, for example, a spray dryer or a drum dryer may be used, resulting in obtaining a powdery catalyst precursor. Alternatively, the mixed liquid of starting materials may be heated using a box dryer, a tunnel dryer or the like, while flowing an air, to obtain a blockish or flaky catalyst precursor. Furthermore, it is also possible to employ a method of concentrating and evaporating the mixed liquid of starting materials to dryness to give a cake-like solid matter and further heating the solid matter as described above. For the drying method by pressure reduction, for example, a vacuum dryer may be used, thereby obtaining a blockish or flaky catalyst precursor.

The obtained catalyst precursor may be gone through a pulverization step and a classification step for giving a powder having an appropriate particle size, as required, and then sent to a subsequent forming step. In some cases, the catalyst precursor may be once calcinated and then sent to the forming step. A particle size of the catalyst precursor before being sent to the forming step is not particularly limited; however, the catalyst precursor preferably has a particle size such that 90 mass % or more of the particles pass though a sieve with a mesh size of 500 $\mu$m, in view of obtaining excellent formability.

In the forming step, a compact may be obtained by forming the catalyst precursor into a specific shape by extrusion molding, tablet compression, or the like. Alternatively, a supported catalyst may be obtained by making any inert carrier having a specific shape support the mixed liquid of starting materials or the catalyst precursor as the catalytic active component (supporting method).

A shape of the compact obtained by extrusion molding, tablet compression or the like is not particularly limited, and any shape such as a spherical shape, a cylindrical shape, a ring shape, an indeterminate shape or the like, may be taken. Of course, the spherical shape does not have to be a true sphere and a substantially spherical shape will suffice. The same applies also to the cylindrical shape and the ring shape.

As the supporting method, an evaporation to dryness method in which the mixed liquid of starting materials, which is not dried and remains in the form of an aqueous solution or an aqueous slurry, is applied or attached to an inert carrier having a specific shape while heating, and then dried to make the inert carrier support the mixed liquid of starting materials, may be employed, or a granulation method in which an inert carrier is made to support a powdery catalyst precursor may be employed. Among them, the granulation method for making an inert carrier support the catalytic active component with using a centrifugal fluidizing coating method described in Japanese Unexamined Patent Application Publication No. 63-200839, a tumbling granulation method described in Japanese Unexamined Patent Application Publication No. 8-299797, or a rocking mixer method described in Japanese Unexamined Patent Application Publication No. 2004-136267, is particularly preferred. A material and shape of the inert carrier which can be used for the supporting method is explained above.

In the forming step, a forming auxiliary agent for enhancing moldability, a binder, a pore-forming agent for forming appropriate pores in a catalyst, or the like may be added. Specific examples of these agents include organic compounds such as ethylene glycol, glycerin, propionic acid, maleic acid, benzyl alcohol, propyl alcohol, butyl alcohol, phenol and the like; water; and inorganic salts such as nitric acid, ammonium nitrate, ammonium carbonate and the like.

In addition, a strengthening agent such as silica, alumina, glass fiber, silicon carbide, silicon nitride and the like can be used for the purpose of enhancing mechanical strength of the catalyst. The strengthening agent may be added to the mixed liquid of starting materials or may be mixed with the catalyst precursor.

The compact or the supported catalyst which have been obtained in the forming step is sent to a subsequent calcination step, where the compact or the supported catalyst is calcinated. The calcination temperature is preferably in the range of 350°C or more and 600°C or less, and more preferably in the range of 400°C or more and 550°C or less. The calcination period is preferably 1 hour or longer and 10 hours or shorter. A calcinating furnace used for the calcination step is not restricted, and a box furnace, a tunnel furnace or the like, which are generally used, may be used.

In the catalyst of the present invention, the crystallinity T is within the specific range, and further, the crystallinity ratio R is preferably within the specific range. For obtaining such a catalyst, the following method is to be adopted. Thus, in the above preparation method of a catalyst, upon preparation of the mixed liquid of starting materials, an aqueous solution (solution B) containing cobalt and bismuth is added to an aqueous solution (solution A) containing molybdenum over a period of 30 seconds to 10 minutes, preferably 1 minute to 5 minutes. The temperature of the mixed liquid upon mixing the raw materials may be appropriately determined according to the raw materials to be fed. In the above method, it is possible to change the crystallinity ratio R by appropriately varying the composition ratio among molybdenum, bismuth and cobalt in the catalytic active component.

A process for producing acrolein and/or acrylic acid of the present invention is hereinafter explained. The process for producing acrolein and/or acrylic acid of the present invention is a process for producing acrolein and/or acrylic acid by gas-phase catalytic oxidation of propylene with molecular oxygen-containing gas, comprising the step of conducting the gas-phase catalytic oxidation in the presence of the catalyst of the present invention. According to the process for producing acrolein and/or acrylic acid of the present invention, it is possible to stably produce acrolein and/or acrylic acid at a high yield for a long period.

In the process for producing acrolein and/or acrylic acid of the present invention, a reactor to be used is not restricted as long as gas-phase catalytic oxidation is conducted by using the catalyst of the present invention. Any reactor such as a fixed-bed reactor, a fluidized-bed reactor, and a moving bed reactor can be used as the reactor. Preferably, a fixed-bed reactor is used as the reactor.

As the fixed-bed reactor, a tubular reactor is preferable and a multitubular reactor is more preferable. In this case, the fixed-bed reactor comprises one or more of reaction tube(s), in which the catalyst is filled. The reaction tube is generally placed so as to be vertically-oriented in the reactor. An inner diameter of the reaction tube is not particularly limited as long as the catalyst can be filled therein; however, it is preferably in the range of 15 mm or more and 50 mm or less, more preferably 20 mm or more and 40 mm or less, and even more preferably 22 mm or more and 38 mm or less.

The catalyst to be filled in the reaction tube may be one kind or may be two or more kinds; however, the catalyst is preferably filled in the reaction tube preferably to define a plurality of reaction zones, which are provided by dividing the catalyst layer filled in the reaction tube into two or more zones in an axis direction of the reaction tube, such that the catalysts filled in the respective reaction zones have the different crystallinity ratio R from each other. Thus, it is preferable that the reaction tube has a plurality of reaction zones divided in the axis direction of the reaction tube, and the respective reaction zones are filled with the catalysts having the different crystallinity ratio R from each other. More preferably, the catalyst is filled in the reaction tube such that the crystallinity ratios R of the plurality of the reaction zones decrease in an order from an inlet to an outlet of a raw material gas containing propylene and molecular oxygen-containing gas. In this case, the catalysts filled in the respective reaction zones preferably have the crystallinity ratio R in the range of 0.03 or more and 0.20 or less. Further, the above catalyst filling manner may be combined with the following manner: the catalyst is filled such that the volume occupied by the catalyst decreases from the inlet to the outlet of the raw material gas; or a part of the catalyst is diluted with an inert carrier or the like.

A number of the reaction zone is determined appropriately depending on reaction conditions or a scale of the reactor. However, from an industrial viewpoint, the number of the reaction zones is preferably about 2 or more and about 6 or less, because the excessively large number of the reaction zones will cause problems such as complication of a filling operation of the catalyst.

Reaction conditions in the process for producing acrolein and/or acrylic acid of the present invention is not particularly limited; and any reaction condition generally used in this kind of reaction can be adopted. For example, a mixed gas, composed of 1 to 15 volume % (preferably, 4 to 12 volume %) of propylene, 0.5 to 25 volume % (preferably, 2 to 20 volume %) of molecular oxygen, 0 to 30 volume % (preferably, 0 to 25 volume %) of steam, and inert gas such as nitrogen and the like as the residue, may be used as the raw material gas. The raw material gas may be brought into contact with the catalyst at a temperature in the range of 280°C to 450°C, under a pressure in the range of 0.1MPa to 1.0 MPa, at a space velocity (STP) in the range of 300 h$^{-1}$ to 5,000 h$^{-1}$.

EXAMPLES

The present invention will hereinafter be described more specifically by reference to Examples; however, the present invention is not limited to these Examples. The present invention can be put into practice after appropriate modifications or variations within a range meeting the gist of the present invention, all of which are included in the technical scope of the present invention. Hereinafter, the term "part(s) by mass" may be described simply as "part(s)" for convenience sake.

Conversion rate of propylene and a sum of yields of acrolein and acrylic acid were determined by the following equations:

$$\text{Conversion rate of propylene (mol\%)} = (\text{molar quantity of reacted propylene}) / (\text{molar quantity of fed propylene}) \times 100$$

$$\text{Yield of acrolein} + \text{acrylic acid (mol\%)} = (\text{molar quantities of produced acrolein and acrylic acid}) / (\text{molar quantity of fed propylene}) \times 100$$

An X-ray diffraction measurement was conducted using X'PertPro manufactured by Spectris Co., Ltd. A sample for the X-ray diffraction measurement was produced by passing a catalytic active component through a sieve with a mesh size of 150 $\mu$m and compression-forming about 0.5 g of the powder that passed through the sieve into tablets each having a diameter of 16 mm and a thickness of 2 mm. The sample for the X-ray diffraction measurement was used to measure the X-ray diffraction intensity with Cu-K$\alpha$ radiation in the 2$\theta$ range of 5° to 90° under conditions of an X-ray output of 40 mA and 45 kV, an X-ray radiation width of 15 mm, and a measurement temperature of 25°C. From the obtained X-ray diffraction profile, the crystallinity T and the crystallinity ratio R were obtained, following the method according to JIS K-0131 (an absolute method).

(1) Experiment Example 1

(1-1) Preparation of catalyst

3000 parts of distilled water was stirred with the temperature maintained at 60°C, and 300 parts of ammonium paramolybdate, and 1.7 parts of potassium nitrate were dissolved therein to give a solution A. Separately, 300 parts of distilled water was stirred with the temperature maintained at 60°C, and 50 parts of 65 mass % of nitric acid, 137 parts of bismuth nitrate, 412 parts of cobalt nitrate, 114 parts of iron nitrate and 206 parts of nickel nitrate were added thereto to give a solution B. The obtained solution B were divided into two so as to be the ratio of 1:1 by mass part to give a solutions B-1 and B-2. While the solution A being stirred, the solution B-1 was added to the solution A over 2 minutes, and the obtained mixture was stirred for 30 minutes. Subsequently, the solution B-2 was added thereto over 2 minutes, and then stirred for 30 minutes to give a suspended liquid. 200 parts of ammonium paramolybdate was further added to the suspension liquid, and the obtained mixture was stirred for 30 minutes to give a mixed liquid of starting materials. The mixed liquid of starting materials was evaporated to give a cake-like solid matter, and the solid matter was dried for about 5 hours at 200°C under an air atmosphere. The dried solid matter was pulverized to a particle diameter of 250 μm or less to give a powdery catalyst precursor. Into a tumbling granulator, 500 parts of a silica-alumina spherical carrier having an average particle diameter of 4.5 mm was fed and then the catalyst precursor was fed together with a 15 mass % ammonium nitrate aqueous solution as a binder to make the carrier support the catalyst precursor. Then, the obtained supported catalyst precursor was calcinated at 470°C for 6 hours under an air atmosphere to give a catalyst 1. The supported ratio of the catalyst 1 was 100 mass %, and the catalyst 1 had the following metal element composition excluding oxygen.

Catalyst 1 :        $Mo_{12}Bi_{1.2}Co_6Fe_{1.2}Ni_3K_{0.07}$

The supported ratio was determined by the following equations:

$$\text{Supported ratio (mass\%)} = (\text{mass of a catalyst (g)} - \text{mass of a used carrier (g)}) / (\text{mass of a used carrier (g)}) \times 100$$

In the catalyst 1, the crystallinity T in the 2θ range of 5° to 90°, measured by an X-ray diffraction analysis, of the catalytic active component was 7.1 %, and the crystallinity ratio R was 0.10.

(1-2) Reaction

A reactor having a reaction tube (reaction tube length: 3,000 mm, inner diameter: 25 mm) made of stainless steel and a shell in which a heat medium is flowed and which covers the reaction tube was vertically-installed. The catalyst 1 was filled into the reactor by dropping the catalyst 1 from the top of the reaction tube so that the layer length comes to be 2900 mm. While maintaining the temperature of the heat medium (a reaction temperature) at 310°C, a mixed gas consisting of 6 volume % of propylene, 11 volume % of oxygen, 15 volume % of steam and an inert gas such as nitrogen as the residue was introduced from the bottom of the reaction tube filled with the catalyst at a space velocity (STP) of 1650 hr$^{-1}$, thereby conducting oxidation reaction of propylene. The results of the conversion rate of propylene and the yield of acrolein + acrylic acid are shown in Table 1. Further, the result after continuous reaction for 4000 hours, that was conducted while adjusting the reaction temperature appropriately, is also shown in Table 1. In Experiment Example 1, the initial yield was as high as 92.8 mol%, and the decrease of the yield after 4000 hours was suppressed to be only in the amount of 0.2 mol%. In Experiment Example 1, acrolein and acrylic acid were produced stably at a high yield for a long period.

(2) Experiment Example 2 (Comparative Example)

A catalyst 2 was prepared in the same manner as in Experiment Example 1, except that the maintaining temperature of the solutions A and B were changed to 70°C, the amounts of ammonium paramolybdate added to the solution A and the suspension liquid were changed to 500 parts and 0 part, respectively, the solution B was not divided into two, and the period for adding the solution B to the solution A was changed to within 10 seconds in Experiment Example 1. The supported ratio of the catalyst 2 was 100 mass %, and the metal element composition of the catalyst 2 was the same as that of the catalyst 1. In the catalyst 2, the crystallinity T was 18.9% and the crystallinity ratio R was 0.02.
The obtained catalyst 2 was filled in the same manner as in Experiment Example 1 and oxidation reaction of propylene was conducted under the same conditions as in Experiment Example 1. The results are shown in Table 1. Further, the result after continuous reaction for 4000 hours, that was conducted while adjusting the reaction temperature appropriately, is also shown in Table 1. In Experiment Example 2, since the crystallinity T of the catalyst 2 was more than 18% and the crystallinity ratio R of the catalyst 2 was a low value of 0.02, the initial yield was as low as 90.7 mol%. After 4000 hours, the yield further decreased, and the amount of the decrease of the yield was as much as 1.5 mol% compared to the initial stage.

(3) Experiment Example 3

A catalyst 3 was prepared in the same manner as in Experiment Example 1, except that the maintaining temperature of the solutions A and B were changed to 70°C, the amounts of ammonium paramolybdate added to the solution A and the suspension liquid were changed to 500 parts and 0 part, respectively, the amounts of bismuth nitrate, cobalt nitrate, iron nitrate and nickel nitrate were changed to 206 parts, 481 parts, 191 parts and 103 parts, respectively, and the periods for adding the solutions B-1 and B-2 to the solution A were respectively changed to 1 minute in Experiment Example 1. The supported ratio of the catalyst 3 was 100 mass %, and the catalyst 3 had the following metal element composition excluding oxygen.

Catalyst 3 : $Mo_{12}Bi_{1.8}Co_7Fe_2Ni_{1.5}K_{0.07}$

In the catalyst 3, the crystallinity T was 14.3% and the crystallinity ratio R was 0.06.

The obtained catalyst 3 was filled in the same manner as in Experiment Example 1 and oxidation reaction of propylene was conducted under the same conditions as in Experiment Example 1. The results are shown in Table 1. In Experiment Example 3, the initial yield was as high as 93.0 mol%.

(4) Experiment Example 4 (Comparative Example)

A catalyst 4 was prepared in the same manner as in Experiment Example 1, except that the amounts of ammonium paramolybdate added to the solution A and the suspension liquid were changed to 100 parts and 400 parts, respectively, the amounts of bismuth nitrate, cobalt nitrate, nickel nitrate and potassium nitrate were changed to 172 parts, 343 parts, 137 parts and 1.2 parts, respectively, the solution B was not divided into two, the period for adding the solution B to the solution A was changed to 12 minutes, and the amount of silica-alumina spherical carrier fed into the tumbling granulator was changed to 450 parts in Experiment Example 1. The supported ratio of the catalyst 4 was 100 mass %, and the catalyst 4 had the following metal element composition excluding oxygen.

Catalyst 4 : $Mo_{12}Bi_{1.5}Co_5Fe_{1.2}Ni_2K_{0.05}$

In the catalyst 4, the crystallinity T was 4.5% and the crystallinity ratio R was 0.21.

The obtained catalyst 4 was filled in the same manner as in Experiment Example 1 and oxidation reaction of propylene was conducted under the same conditions as in Experiment Example 1. The results are shown in Table 1. Further, the result after continuous reaction for 4000 hours, that was conducted while adjusting the reaction temperature appropriately, is also shown in Table 1. In Experiment Example 4, since the catalyst 4 had a somewhat low crystallinity T of 4.5% and a high crystallinity ratio R of 0.21, the yield was lower than those in Experiment Examples 1 and 3, that is, 91.7 mol% in the initial stage and 90.9 mol% after 4000 hours.

(5) Experiment Example 5 (Comparative Example)

A catalyst 5 was prepared in the same manner as in Experiment Example 4, except that the amounts of ammonium paramolybdate added to the solution A and the suspension liquid were changed to 0 part and 500 parts, respectively, and the period for adding the solution B to the solution A was changed to 20 minutes in Experiment Example 4. The supported ratio of the catalyst 5 was 100 mass %, and the metal element composition of the catalyst 5 was the same as that of the catalyst 4. In the catalyst 5, the crystallinity T was 3.4% and the crystallinity ratio R was 0.22.

The obtained catalyst 5 was filled in the same manner as in Experiment Example 1 and oxidation reaction of propylene was conducted under the same conditions as in Experiment Example 1. The results are shown in Table 1. Further, the result after continuous reaction for 4000 hours, that was conducted while adjusting the reaction temperature appropriately, is also shown in Table 1. In Experiment Example 5, since the crystallinity T of the catalyst 5 was less than 4% and the crystallinity ratio R of the catalyst 5 was as high as 0.22, the initial yield was resulted in a low value of 89.6 mol%. After 4000 hours, the yield further decreased, and the amount of the decrease of the yield was as much as 1.9 mol% compared to the initial stage.

(6) Experiment Example 6 (Comparative Example)

A catalyst 6 was prepared in the same manner as in Experiment Example 3, except that the amounts of bismuth nitrate and iron nitrate were changed to 183 parts and 114 parts, respectively, and the periods for adding the solutions B-1 and B-2 to the solution A were respectively changed to within 10 seconds in Experiment Example 3. The supported ratio of the catalyst 6 was 100 mass %, and the catalyst 6 had the following metal element composition excluding oxygen.

Catalyst 6 : $Mo_{12}Bi_{1.6}Co_7Fe_{1.2}Ni_{1.5}K_{0.07}$

In the catalyst 6, the crystallinity T was 17.1 % and the crystallinity ratio R was 0.02.

The obtained catalyst 6 was filled in the same manner as in Experiment Example 1 and oxidation reaction of propylene was conducted under the same conditions as in Experiment Example 1. The results are shown in Table 1. In Experiment Example 6, since the catalyst 6 had a somewhat high crystallinity T of 17.1 % and a low crystallinity ratio R of 0.02, the initial yield was lower than those in Experiment Examples 1 and 3, that is, 91.4 mol%.

(7) Experiment Example 7 (Comparative Example)

A catalyst 7 was prepared in the same manner as in Experiment Example 6, except that the maintaining temperature of the solutions A and B were changed to 50°C, the amounts of ammonium paramolybdate added to the solution A and the suspension liquid were changed to 0 part and 500 parts, respectively, and the solution B was not divided into two in Experiment Example 6. The supported ratio of the catalyst 7 was 100 mass %, and the metal element composition of the catalyst 7 was the same as that of the catalyst 6. In the catalyst 7, the crystallinity T was 3.4% and the crystallinity ratio R was 0.01.

The obtained catalyst 7 was filled in the same manner as in Experiment Example 1 and oxidation reaction of propylene was conducted under the same conditions as in Experiment Example 1. The results are shown in Table 1. In Experiment Example 7, since the crystallinity T of the catalyst 7 was less than 4% and the crystallinity ratio R was as low as 0.01, the initial yield was resulted in a low value of 90.4 mol%.

(8) Experiment Example 8

A catalyst 8 was prepared in the same manner as in Experiment Example 4, except that the maintaining temperature of the solutions A and B were changed to 70°C, the amounts of ammonium paramolybdate added to the solution A and the suspension liquid were changed to 400 parts and 100 parts, respectively, the amounts of bismuth nitrate and iron nitrate were changed to 145 parts and 95.4 parts, respectively, nickel nitrate was not added, the period for adding the solution B to the solution A was changed to 30 seconds, and the amount of silica-alumina spherical carrier fed into the tumbling granulator was changed to 430 parts in Experiment Example 4. The supported ratio of the catalyst 8 was 100 mass %, and the catalyst 8 had the following metal element composition excluding oxygen.

$$\text{Catalyst 8 :} \qquad Mo_{12}Bi_{1.3}Co_5Fe_1K_{0.05}$$

In the catalyst 8, the crystallinity T was 9.7% and the crystallinity ratio R was 0.04.

The obtained catalyst 8 was filled in the same manner as in Experiment Example 1 and oxidation reaction of propylene was conducted under the same conditions as in Experiment Example 1. The results are shown in Table 1. In Experiment Example 8, the initial yield was as high as 92.6 mol%.

(9) Experiment Example 9

A catalyst 9 was prepared in the same manner as in Experiment Example 1, except that the maintaining temperature of the solutions A and B were changed to 70°C, the amounts of ammonium paramolybdate added to the solution A and the suspension liquid were changed to 350 parts and 150 parts, respectively, the amounts of bismuth nitrate, cobalt nitrate and iron nitrate were changed to 229 parts, 481 parts and 143 parts, nickel nitrate was not added, and the periods for adding the solutions B-1 and B-2 to the solution A were respectively changed to 1 minute in Experiment Example 1. The supported ratio of the catalyst 9 was 100 mass %, and the catalyst 9 had the following metal element composition excluding oxygen.

$$\text{Catalyst 9 :} \qquad Mo_{12}Bi_2Co_7Fe_{1.5}K_{0.07}$$

In the catalyst 9, the crystallinity T was 7.9% and the crystallinity ratio R was 0.12.

The obtained catalyst 9 was filled in the same manner as in Experiment Example 1 and oxidation reaction of propylene was conducted under the same conditions as in Experiment Example 1. The results are shown in Table 1. In Experiment Example 9, the initial yield was as high as 93.0 mol%.

(10) Experiment Example 10

A catalyst 10 was prepared in the same manner as in Experiment Example 1, except that the amounts of ammonium paramolybdate added to the solution A and the suspension liquid were changed to 0 part and 500 parts, respectively, the amounts of bismuth nitrate, cobalt nitrate, iron nitrate and potassium nitrate were changed to 80.1 parts, 378 parts, 143 parts and 1.2 parts, nickel nitrate was not added, the periods for adding the solutions B-1 and B-2 to the solution A were respectively changed to 4 minutes, and the amount of silica-alumina spherical carrier fed into the tumbling granulator was changed to 430 parts in Experiment Example 1. The supported ratio of the catalyst 10 was 100 mass %, and the catalyst 10 had the following metal element composition excluding oxygen.

Catalyst 10 :     $Mo_{12}Bi_{0.7}Co_{5.5}Fe_{1.5}K_{0.05}$

In the catalyst 10, the crystallinity T was 5.9% and the crystallinity ratio R was 0.14.

The obtained catalyst 10 was filled in the same manner as in Experiment Example 1 and oxidation reaction of propylene was conducted under the same conditions as in Experiment Example 1. The results are shown in Table 1. In Experiment Example 10, the initial yield was a relatively high value of 92.2 mol%.

(11) Experiment Example 11

A catalyst 11 was prepared in the same manner as in Experiment Example 6, except that the amounts of bismuth nitrate, cobalt nitrate, iron nitrate and nickel nitrate were changed to 229 parts, 412 parts, 172 parts and 137 parts, and the periods for adding the solutions B-1 and B-2 to the solution A were respectively changed to 30 seconds in Experiment Example 6. The supported ratio of the catalyst 11 was 104 mass %, and the catalyst 11 had the following metal element composition excluding oxygen.

Catalyst 11 :     $Mo_{12}Bi_2Co_6Fe_{1.8}Ni_2K_{0.07}$

In the catalyst 11, the crystallinity T was 16.2% and the crystallinity ratio R was 0.03.

The obtained catalyst 11 was filled in the same manner as in Experiment Example 1 and oxidation reaction of propylene was conducted under the same conditions as in Experiment Example 1. The results are shown in Table 1. In Experiment Example 11, the initial yield was a relatively high value of 92.2 mol%.

[Table 1]

| | Catalyst No. | Crystallinity T(%) | Crystallinity Ratio R | Passage of Time (hours) | Reaction Temperature (°C) | Conversion Rate of Propylene (mol%) | Yield of Acrylic Acid + Acrolein (mol%) |
|---|---|---|---|---|---|---|---|
| Experiment Example 1 | 1 | 7.1 | 0.10 | initial | 310 | 98.4 | 92.8 |
| | | | | 4000 | 314 | 98.4 | 92.6 |
| Experiment Example 2 | 2 | 18.9 | 0.02 | initial | 310 | 97.5 | 90.7 |
| | | | | 4000 | 326 | 97.3 | 89.2 |
| Experiment Example 3 | 3 | 14.3 | 0.06 | initial | 310 | 98.5 | 93.0 |
| Experiment Example 4 | 4 | 4.5 | 0.21 | initial | 310 | 97.8 | 91.7 |
| | | | | 4000 | 317 | 97.7 | 90.9 |
| Experiment Example 5 | 5 | 3.4 | 0.22 | initial | 310 | 96.6 | 89.6 |
| | | | | 4000 | 320 | 96.4 | 87.5 |
| Experiment Example 6 | 6 | 17.1 | 0.02 | initial | 310 | 97.7 | 91.4 |
| Experiment Example 7 | 7 | 3.4 | 0.01 | initial | 310 | 97.1 | 90.4 |
| Experiment Example 8 | 8 | 9.7 | 0.04 | initial | 310 | 98.0 | 92.6 |
| Experiment Example 9 | 9 | 7.9 | 0.12 | initial | 310 | 98.4 | 93.0 |
| Experiment Example 10 | 10 | 5.9 | 0.14 | initial | 310 | 98.1 | 92.2 |

(continued)

| | Catalyst No. | Crystallinity T(%) | Crystallinity Ratio R | Passage of Time (hours) | Reaction Temperature (°C) | Conversion Rate of Propylene (mol%) | Yield of Acrylic Acid + Acrolein (mol%) |
|---|---|---|---|---|---|---|---|
| Experiment Example 11 | 11 | 16.2 | 0.03 | initial | 310 | 98.2 | 92.2 |

(12) Experiment Example 12

(12-1) Preparation of catalyst

A catalyst 12 was prepared in the same manner as in Experiment Example 1, except that the carrier used in Experiment Example 1 was changed to a silica-alumina spherical carrier having an average particle diameter of 6.5 mm. The supported ratio of the catalyst 12 was 100 mass %, and the metal element composition, the crystallinity T and the crystallinity ratio R of the catalyst 12 were the same as those of the catalyst 1.

(12-2) Reaction

A reactor having a reaction tube (reaction tube length: 3,000 mm, inner diameter: 25 mm) made of stainless steel and a shell in which a heat medium is flowed and which covers the reaction tube was vertically-installed. The catalysts 12 and 3 were filled into the reactor by sequentially dropping the catalyst 12 and the catalyst 3 from the top of the reaction tube so that the layer lengths come to be 800 mm for the catalyst 12 and 2100 mm for the catalyst 3.

While maintaining the temperature of the heat medium (a reaction temperature) at 315°C, a mixed gas consisting of 8 volume % of propylene, 15 volume % of oxygen, 8 volume % of steam and an inert gas such as nitrogen as the residue was introduced from the bottom of the reaction tube filled with the catalysts at a space velocity (STP) of 1950 $hr^{-1}$, thereby conducting oxidation reaction of propylene. In Experiment Example 12, the catalyst 12 was placed on an inlet side, with respect to the flow of the mixed gas, of the reaction tube, and the catalyst 3 was placed on an outlet side, with respect to the flow of the mixed gas, of the reaction tube. The reaction was conducted continuously for 4000 hours while adjusting the reaction temperature appropriately. The results are shown in Table 2. In Experiment Example 12, two kinds of catalysts, i.e. the catalysts 3 and 12, each of which has the crystallinity ratio R in the range of 0.03 or more and 0.20 or less, were filled into the reaction tube. The catalyst 12 having the higher crystallinity ratio R was placed on the inlet side, and the catalyst 3 having the lower crystallinity ratio R was placed on the outlet side. As a result, the yield was a high value of 92.5 mol% or more both in the initial stage and after 4000 hours.

(13) Experiment Example 13

A catalyst 13 was prepared in the same manner as in Experiment Example 3, except that the carrier used in Experiment Example 3 was changed to a silica-alumina spherical carrier having an average particle diameter of 6.5 mm. The supported ratio of the catalyst 13 was 100 mass %, and the metal element composition, the crystallinity T and the crystallinity ratio R of the catalyst 13 were the same as those of the catalyst 3.

Into the same reaction tube as in the Experiment Example 12, the catalysts 13 and 1 were filled by sequentially dropping the catalyst 13 and the catalyst 1 from the top of the reaction tube so that the layer lengths come to be 800 mm for the catalyst 13 and 2100 mm for the catalyst 1, and oxidation reaction of propylene was conducted under the same conditions as in Experiment Example 12. In Experiment Example 13, the catalyst 13 was placed on the inlet side, with respect to the flow of the mixed gas, of the reaction tube, and the catalyst 1 was placed on the outlet side, with respect to the flow of the mixed gas, of the reaction tube. The results are shown in Table 2. In Experiment Example 13, two kinds of catalysts, i.e. the catalysts 1 and 13, each of which has the crystallinity ratio R in the range of 0.03 or more and 0.20 or less, were filled into the reaction tube. The catalyst 13 having the lower crystallinity ratio R was placed on the inlet side, and the catalyst 1 having the higher crystallinity ratio R was placed on the outlet side. Also in Experiment Example 13, the yield was maintained to be high both in the initial stage and after 4000 hours; however, the performance in Experiment Example 13 was lower than that in Experiment Example 12.

(14) Experiment Example 14 (Comparative Example)

A catalyst 14 was prepared in the same manner as in Experiment Example 2, except that the carrier used in Experiment Example 2 was changed to a silica-alumina spherical carrier having an average particle diameter of 6.5 mm. The supported ratio of the catalyst 14 was 100 mass %, and the metal element composition, the crystallinity T and the crystallinity ratio R of the catalyst 14 were the same as those of the catalyst 2.

Into the same reaction tube as in the Experiment Example 12, the catalysts 14 and 2 were filled by sequentially dropping the catalyst 14 and the catalyst 2 from the top of the reaction tube so that the layer lengths come to be 800 mm for the catalyst 14 and 2100 mm for the catalyst 2, and oxidation reaction of propylene was conducted under the same conditions as in Experiment Example 12. In Experiment Example 14, the catalyst 14 was placed on the inlet side, with respect to the flow of the mixed gas, of the reaction tube, and the catalyst 2 was placed on the outlet side, with respect to the flow of the mixed gas, of the reaction tube. The results are shown in Table 2. In Experiment Example 14, the catalysts 2 and 14, which have the same crystallinity ratio R each other out of the range of 0.03 to 0.20, were filled into the reaction tube. The yield in Experiment Example 14 was lower than those in the Experiment Examples 12 and 13.

(15) Experiment Example 15 (Comparative Example)

A catalyst 15 was prepared in the same manner as in Experiment Example 5, except that the carrier used in Experiment Example 5 was changed to a silica-alumina spherical carrier having an average particle diameter of 6.5 mm. The supported ratio of the catalyst 15 was 100 mass %, and the metal element composition, the crystallinity T and the crystallinity ratio R of the catalyst 15 were the same as those of the catalyst 5.

Into the same reaction tube as in the Experiment Example 12, the catalysts 15 and 2 were filled by sequentially dropping the catalyst 15 and the catalyst 2 from the top of the reaction tube so that the layer lengths come to be 800 mm for the catalyst 15 and 2100 mm for the catalyst 2, and oxidation reaction of propylene was conducted under the same conditions as in Experiment Example 12. In Experiment Example 15, the catalyst 15 was placed on the inlet side, with respect to the flow of the mixed gas, of the reaction tube, and the catalyst 2 was placed on the outlet side, with respect to the flow of the mixed gas, of the reaction tube. The results are shown in Table 2. In Experiment Example 15, two kinds of catalysts, i.e. the catalysts 2 and 15, each of which has the crystallinity ratio R out of the range of 0.03 to 0.20, were filled into the reaction tube. The catalyst 15 having the higher crystallinity ratio R was placed on the inlet side, and the catalyst 2 having the lower crystallinity ratio R was placed on the outlet side. The yield in Experiment Example 15 was lower than those in the Experiment Examples 12 and 13.

[Table 2]

|  |  | Passage of Time (hours) | Reaction Temperature (°C) | Conversion Rate of Propylene (mol%) | Yield of Acrylic Acid + Acrolein (mol%) |
|---|---|---|---|---|---|
| Experiment Example 12 | inlet side : catalyst 12 (R=0.10) | initial | 315 | 98.3 | 92.8 |
|  | outlet side : catalyst 3 (R=0.06) | 4000 | 320 | 98.3 | 92.5 |
| Experiment Example 13 | inlet side : catalyst 13 (R=0.06) | initial | 315 | 98.1 | 92.4 |
|  | outlet side : catalyst 1 (R=0.10) | 4000 | 321 | 98.0 | 92.1 |
| Experiment Example 14 | inlet side : catalyst 14 (R=0.02) | initial | 315 | 97.2 | 89.6 |
|  | outlet side : catalyst 2 (R=0.02) | 4000 | 335 | 96.9 | 88.1 |

(continued)

|  |  | Passage of Time (hours) | Reaction Temperature (°C) | Conversion Rate of Propylene (mol%) | Yield of Acrylic Acid + Acrolein (mol%) |
|---|---|---|---|---|---|
| Experiment Example 15 | inlet side : catalyst 15 (R=0.22) | initial | 315 | 97.4 | 89.8 |
|  | outlet side : catalyst 2 (R=0.02) | 4000 | 334 | 97.2 | 88.2 |

**Claims**

1. A catalyst for producing acrolein and/or acrylic acid from propylene by gas-phase catalytic oxidation with molecular oxygen-containing gas, comprising a catalytic active component, wherein:

   the catalyst is prepared by a method comprising the step of adding an aqueous solution containing cobalt and bismuth to an aqueous solution containing molybdenum over a period of 30 seconds to 10 minutes; the catalytic active component has a composition expressed by the following formula (4); and the catalytic active component contains a crystalline part showing a peak at $2\theta = 26.5 \pm 0.5°$, measured by an X-ray diffraction analysis with Cu-K$\alpha$ radiation, and has a crystallinity T of 4% or more and 18% or less in the $2\theta$ range of 5° to 90°, measured by an X-ray diffraction analysis with Cu-K$\alpha$ radiation; and a crystallinity ratio R expressed by the following formula (1) is in the range of 0.03 or more and 0.20 or less

$$R = M/T \qquad (1)$$

   wherein M represents a crystallinity of the peak at $2\theta = 26.5 \pm 0.5°$ in the catalytic active component, measured by the X-ray diffraction analysis with Cu-K$\alpha$ radiation,

$$MO_aBi_bCo_cX1_dX2_eX3_fX4_gO_z \qquad (4)$$

   wherein Mo is molybdenum; Bi is bismuth; Co is cobalt; X1 represents at least one kind of element selected from the group consisting of iron and nickel; X2 represents at least one kind of element selected from the group consisting of alkali metals, alkali earth metals, boron and thallium; X3 represents at least one kind of element selected from the group consisting of tungsten, silicon, aluminum, zirconium and titanium; X4 represents at least one kind of element selected from the group consisting of phosphorus, tellurium, antimony, tin, cerium, lead, niobium, manganese, arsenic and zinc; O is oxygen; a, b, c, d, e, f, g and z mean atomic ratios of Mo, Bi, Co, X1, X2, X3, X4 and O, respectively, and meet inequalities: $a = 12$, $0.1 \leq b \leq 10$, $0.1 \leq c \leq 20$, $0.1 \leq d \leq 20$, $0.001 \leq e \leq 10$, $0 \leq f \leq 30$ and $0 \leq g \leq 10$, respectively; and z is a numeral value determined by oxidation states of respective elements.

2. The catalyst for producing acrolein and/or acrylic acid according to claim 1, further comprising an inert carrier for supporting the catalytic active component.

3. A process for producing acrolein and/or acrylic acid by gas-phase catalytic oxidation of propylene with molecular oxygen, comprising the step of conducting the gas-phase catalytic oxidation in the presence of the catalyst according to claim 1 or 2.

4. The process for producing acrolein and/or acrylic acid according to claim 3, wherein the catalyst is filled in a reaction tube of a fixed-bed reactor.

5. The process for producing acrolein and/or acrylic acid according to claim 4, wherein the reaction tube has a plurality of reaction zones divided in an axis direction of the reaction tube, and

the respective reaction zones are filled with the catalysts having the different crystallinity ratio R from each other.

6. The process for producing acrolein and/or acrylic acid according to claim 5,
wherein
the crystallinity ratios R of the plurality of the reaction zones decreases in an order from an inlet to an outlet of a raw material gas containing propylene and molecular oxygen-containing gas.

**Patentansprüche**

1. Katalysator zur Herstellung von Acrolein und/oder Acrylsäure aus Propylen durch katalytische Gasphasenoxidation mit molekularem Sauerstoff enthaltenden Gas, umfassend eine katalytisch aktive Komponente, wobei:

der Katalysator durch ein Verfahren hergestellt ist, umfassend den Schritt des Hinzufügens einer wässrigen Lösung, die Cobalt und Bismut enthält, zu einer wässrigen Lösung, die Molybdän enthält, über einen Zeitraum von 30 Sekunden bis 10 Minuten;
die katalytisch aktive Komponente eine Zusammensetzung aufweist, ausgedrückt durch die folgende Formel (4); und
die katalytisch aktive Komponente einen kristallinen Bestandteil enthält, der einen Peak bei $2\theta = 26{,}5 \pm 0{,}5°$ zeigt, gemessen durch eine Röntgenbeugungsanalyse mit Cu-K$\alpha$-Strahlung, und eine Kristallinität T von 4% oder mehr und 18% oder weniger im $2\theta$ Bereich von 5° bis 90° aufweist, gemessen durch eine Röntgenbeugungsanalyse mit Cu-K$\alpha$-Strahlung; und
ein Kristallinitätsverhältnis R, ausgedrückt durch die folgende Formel (1), in dem Bereich von 0,03 oder mehr und 0,20 oder weniger ist

$$R = M/T \qquad (1)$$

wobei M eine Kristallinität des Peaks bei $2\theta = 26{,}5 \pm 0{,}5°$ in der katalytisch aktiven Komponente darstellt, gemessen durch die Röntgenbeugungsanalyse mit Cu-K$\alpha$-Strahlung,

$$Mo_aBi_bCo_cX1_dX2_eX3_fX4_gO_z \qquad (4)$$

wobei Mo Molybdän ist; Bi Bismut ist; Co Cobalt ist; X1 mindestens eine Art von Elementen darstellt, ausgewählt aus der Gruppe, bestehend aus Eisen und Nickel; X2 mindestens eine Art von Elementen darstellt, ausgewählt aus der Gruppe, bestehend aus Alkalimetallen, Erdalkalimetallen, Bor und Thallium; X3 mindestens eine Art von Elementen darstellt, ausgewählt aus der Gruppe, bestehend aus Wolfram, Silicium, Aluminium, Zirconium und Titan; X4 mindestens eine Art von Elementen darstellt, ausgewählt aus der Gruppe, bestehend aus Phosphor, Tellur, Antimon, Zinn, Cer, Blei, Niob, Mangan, Arsen und Zink; O Sauerstoff ist; a, b, c, d, e, f, g und z jeweils Atomverhältnisse von Mo, Bi, Co, X1, X2, X3, X4 und O bedeuten und die Ungleichungen: $a = 12$, $0{,}1 \leq b \leq 10$, $0{,}1 \leq c \leq 20$, $0{,}1 \leq d \leq 20$, $0{,}001 \leq e \leq 10$, $0 \leq f \leq 30$ and $0 \leq g \leq 10$ erfüllen; und z ein numerischer Wert ist, bestimmt durch Oxidationsstufen der jeweiligen Elemente.

2. Katalysator zur Herstellung von Acrolein und/oder Acrylsäure nach Anspruch 1, ferner umfassend einen inerten Träger zur Aufnahme der katalytisch aktiven Komponente.

3. Verfahren zur Herstellung von Acrolein und/oder Acrylsäure durch katalytische Gasphasenoxidation von Propylen mit molekularem Sauerstoff, umfassend den Schritt des Durchführens der katalytischen Gasphasenoxidation in der Gegenwart des Katalysators nach Anspruch 1 oder 2.

4. Verfahren zur Herstellung von Acrolein und/oder Acrylsäure nach Anspruch 3, wobei der Katalysator in ein Reaktionsrohr eines Festbettreaktors eingefüllt ist.

5. Verfahren zur Herstellung von Acrolein und/oder Acrylsäure nach Anspruch 4,
wobei
das Reaktionsrohr eine Mehrzahl an Reaktionsbereichen aufweist, die in einer Längsachsenrichtung des Reaktionsrohres unterteilt sind, und

die jeweiligen Reaktionsbereiche mit den Katalysatoren befüllt sind, die ein voneinander unterschiedliches Kristallinitätsverhältnis R aufweisen.

6. Verfahren zur Herstellung von Acrolein und/oder Acrylsäure nach Anspruch 5,
   wobei
   die Kristallinitätsverhältnisse R der Mehrzahl an Reaktionsbereichen in einer Reihenfolge von einem Einlass zu einem Auslass eines Rohstoffgases, das Propylen und molekularen Sauerstoff enthaltendes Gas enthält, abnehmen.

**Revendications**

1. Catalyseur pour la production d'acroléine et/ou d'acide acrylique à partir de propylène par oxydation catalytique en phase gazeuse avec un gaz contenant de l'oxygène moléculaire, comprenant un composant catalytiquement actif, où :

   le catalyseur est préparé par un procédé comprenant l'étape d'addition d'une solution aqueuse contenant du cobalt et du bismuth à une solution aqueuse contenant le molybdène, sur une période allant de 30 secondes à 10 minutes ;
   le composant catalytiquement actif a une composition exprimée par la formule (4) suivante, et
   le composant catalytiquement actif contient une partie cristalline présentant un pic à $2\theta = 26{,}5 \pm 0{,}5°$, mesuré par analyse de diffraction des rayons X avec un rayonnement Cu-K$\alpha$ il a une cristallinité T de 4% ou plus et 18% ou moins dans la plage $2\theta$ allant de 5° à 90°, mesuré par analyse de diffraction des rayons X avec un rayonnement Cu-K$\alpha$ ; et un rapport de cristallinité R exprimé par la formule (1) suivante se situe dans la plage de 0,03 ou plus et 0,20 ou moins

$$R = M/T \qquad\qquad (1)$$

   où M représente la cristallinité du pic à $2\theta = 26{,}5 \pm 0{,}5°$ dans le composant catalytiquement actif, mesuré par analyse de diffraction des rayons X avec un rayonnement Cu-K$\alpha$

$$MO_aBi_bCo_cX1_dX2_eX3_fX4_gO_z \qquad (4)$$

   où Mo est molybdène ; Bi est bismuth ; Co est cobalt ; X1 représente au moins un type d'élément choisi parmi le groupe consistant en le fer et le nickel ; X2 représente au moins un type d'élément choisi parmi le groupe consistant en des métaux alcalins, des métaux alcalino-terreux, le bore et le thallium ; X3 représente au moins un type d'élément choisi parmi le groupe consistant en le tungstène, le silicium, l'aluminium, le zirconium et le titane ; X4 représente au moins un type d'élément choisi parmi le groupe consistant en le phosphore, le tellure, l'antimoine, l'étain, le cérium, le plomb, le niobium, le manganèse, l'arsenic et le zinc ; O est oxygène ; a, b, c, d, e, f, g et z représentent les rapports atomiques de Mo, Bi, Co, X1, X2, X3, X4 et O, respectivement, et satisfont les inégalités: a=12 ; $0{,}1 \leq b \leq 10$ ; $0{,}1 \leq c \leq 20$ ; $0{,}1 \leq d \leq 20$; $0{,}001 \leq e \leq 10$ ; $0 \leq f \leq 30$ et $0 \leq g \leq 10$, respectivement; et z est une valeur numérique déterminée par les états d'oxydation des éléments respectifs.

2. Catalyseur de production d'acroléine et/ou d'acide acrylique selon la revendication 1, comprenant en outre, un support inerte pour supporter le composant catalytiquement actif.

3. Procédé de production d'acroléine et/ou d'acide acrylique par oxydation catalytique en phase gazeuse du propylène avec l'oxygène moléculaire, comprenant l'étape de réalisation de la réaction d'oxydation catalytique en phase gazeuse en présence du catalyseur selon la revendication 1 ou 2.

4. Procédé de production d'acroléine et/ou d'acide acrylique selon la revendication 3, où le catalyseur est placé dans un tube rédactionnel d'un réacteur à lit fixe.

5. Procédé de production d'acroléine et/ou d'acide acrylique selon la revendication 4, où le tube réactionnel présente une série de zones réactionnelles divisées selon la direction axiale du tube réactionnel, et les zones réactionnelles respectives sont garnies de catalyseurs ayant des rapports de cristallinité R différents les uns des autres.

6. Procédé de production d'acroléine et/ou d'acide acrylique selon la revendication 5, où les rapports de cristallinité R de la série des zones réactionnelles diminuent de l'entrée vers la sortie d'un gaz brut contenant le propylène et un gaz contenant de l'oxygène moléculaire.

**EP 2 329 880 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000169149 A
- JP 56028180 A
- JP 1168344 A
- JP 63137755 A
- EP 1571137 A2
- EP 1714955 A1
- EP 2177500 A1
- JP 63200839 A
- JP 8299797 A
- JP 2004136267 A